# EUROPEAN PATENT APPLICATION

(11) **EP 1 736 770 A1**
(43) Date of publication of application: **27.12.2006**
(21) Application number: 05728881.3
(22) Date of filing: 05.04.2005
(51) Int. Cl.: G01N 33/15, G01N 33/53, G01N 33/574, G01N 33/577

(54) **METHOD OF EXAMINING MALIGNANT TUMOR**

(30) Priority: 16.04.2004 JP 2004121298
(71) Applicant: Okabe, Hidetoshi, Fushima-ku Kyoto-shi, Kyoto 6128017 (JP)
(72) Inventor: Okabe, Hidetoshi, Fushima-ku Kyoto-shi, Kyoto 6128017 (JP)
(74) Representative: Krauss, Jan
(86) International application number: PCT/JP2005/006634
(87) International publication number: WO 2005/100983

(57) **Abstract**

A novel examination method for accurately evaluating the effectiveness of treatment with an anticancer drug for the administration of the anticancer drug targeting a tumor-associated factor receptor is provided. In order to evaluate the effectiveness of trastuzumab (Herceptin^{™}) of an anticancer drug, it was found to be meaningful to examine the expression of MUC4, that is, a substance which interacts with HER2/c-erbB-2 belonging to the epidermal growth factor receptor family on the surface of and/or within the cell membrane. From this fact, in order to treat various cancers with an anticancer drug targeting the tumor-associated factor receptors, it was found to be meaningful to detect previously the expression of intracellular ligands against the receptors. The finding completes the present invention.

## Description

### Technical Field

The present invention relates to an examination method for malignant tumors, specifically for therapeutic strategies, predictions of prognosis and anticipations of therapeutic effects in therapy for inhibiting the receptor function of a cell growth factor receptor by a humanized monoclonal antibody.

### Background Art

Agents for molecular targeting therapy against cancers have attracted the attention to their meanings as new type anticancer drugs in contrast to conventional cellular targeting therapeutic agents. Among them, agents having signal transmission inhibitory actions have especially attracted the attention.

An oncogene, c-erbB-2, encodes a protein (HER2/c-erbB-2) having a molecular weight of 185 kDa and a receptor structure penetrating a cell membrane (non-patent document Nos. 1, 2).HER2/c-erbB-2 is a receptor tyrosine kinase and belongs to the EGFR family to have a similar structure to the human epidermal growth factor receptor (EGFR) gene. The extracellular domain of HER2/c-erbB-2 has a receptor structure, to the extracellular domain of which extracellular ligands bind to activate tyrosine kinase present in the intracellular domain. The activation induces autophosphorylation of HER2/c-erbB-2 and phosphorylation of a substance interacting with HER2/c-erbB-2 within a cell, so that proliferative signals are transmitted from the cell surface to the nucleus, thus it is believed that HER2/c-erbB-2 is involved in proliferation and differentiation of a cell (non-patent document No. 3).HER2/c-erbB-2 protein is being overexpressed in various human tumors. In particular, in breast cancer, patients with overexpressed c-erbB-2 gene or protein thereof show poor prognosis, thus c-erbB-2 gene is one of important factors to diagnose, prognosticate, or decide a therapeutic strategy.

Trastuzumab (Herceptin^{™}) is a humanized monoclonal antibody specifically binding to HER2/c-erbB-2 protein. Trastuzumab (Herceptin^{™}) targets HER2/c-erbB-2 protein-overexpressing tumor cells to specifically bind. The binding inhibits the receptor function of HER2/c-erbB-2 protein, thereby to inhibit signal transmission, effecting in the inhibition of tumor cell proliferation. Therefore, the identification of patients to be treated with trastuzumab (Herceptin^{™}) requires the examination of overexpression of HER2/c-erbB-2 protein or situation of c-erbB-2 gene amplification in cancer tissues (non-patent document No. 4).

Currently, as a method for examining HER2/c-erbB-2 expression, immunohistochemical analysis and fluorescence in situ hybridization (FISH method) are performed.

Evaluation of expression of HER2/c-erbB-2 targets only the stainability and the staining intensity of a tumor cell membrane, and excludes a reaction in cytoplasm to target. Reactivities in the cell membranes are ranked according to the following standard into categories of scores from 0 to 3+.

Score 0: tumor cells having no positive staining in the cell membranes, or those having positive staining in the cell membranes of < 10%.
Score 1+: tumor cells having positive staining in the cell membranes of ≥ 10%, as faint as hardly discernible.
Score 2+: tumor cells having positive staining in the cell membranes of ≥ 10%, from weak to mild perfect.
Score 3+: tumor cells having positive staining in the cell membranes of ≥ 10%, intense perfect.

In evaluation of expression of HER2/c-erbB-2 according to the standard described above, treatment with trastuzumab (Herceptin^{™}) is evaluated in case of 3+ to be effective, but in cases of 2+ to be ambiguous. In summary, treatment with trastuzumab (Herceptin^{™}) is certainly confirmed to be effective in case of 3+, but is not always effective in case of 2+.On the other hand, positive staining given according to FISH method shows that treatment with trastuzumab (Herceptin^{™}) is ensured to be effective at a certain rate.

It has demonstrated that immunohistochemical staining of tissue section is a reliable method to evaluate differences in proteins among different tissues. Immunohistochemical analysis is an examination method for using an antibody as the probe and usually visualizing the antigen present in situ in a cell by a chromogenic or fluorescence method.

The fluorescence in situ hybridization method (FISH method) has recently developed a method for directly evaluating the existence of certain genes within intact cells. FISH method is a method for using DNA or RNA probe labeled with fluorescent dye or hapten, binding to cellular RNAs or DNAs within a cell or tissue section fixed onto a slide glass, and then detecting the genes by fluorescence signals under a fluorescence microscope.

Immunohistochemical analysis and FISH method were compared on effectiveness of examination for HER2/c-erbB-2 expression. As a result, a conclusion was obtained that FISH method is better than immunohistochemical analysis in reproducibility and allows more accurate evaluation of expression state of HER2/c-erbB-2.Further, it is known that the rate of cases effectively treated with trastuzumab (Herceptin^{™}) in the positive cases is higher by the FISH method than by the immunohistochemical analysis. However, as the FISH method is laborious and costly, therefore a conclusion was obtained that the FISH method should be performed to determine effectiveness of treatment with trastuzumab (Herceptin^{™}) only for the cases of 2+ shown by the immunohistochemical analysis (non-patent document No. 5).

Mucin 4 (MUC4) is a substance which binds to HER2/c-erbB-2 within a cell membrane (non-patent article 6).A cell, which expresses HER2/c-erbB-2 and contains no MUC4/sialomucin, is transfected with MUC4/sialomucin to phosphorylate tyrosine at the 1248th of the HER2/c-erbB-2, indicating that MUC4/sialomucin is involved in the phosphorylation of HER2/c-erbB-2 (non-patent article 6).
Non-patent document No.1: Biochem. et Biophys. Acta, 1198, 165-184 (1994)
Non-patent document No. 2: Oncogene, 9, 2109-2123 (1994)
Non-patent document No.3: Brit. J. Cancer, 72, 1259-1266 (1995)
Non-patent document No.4: Science, 235, 177-182 (1987)
Non-patent document No.5: J. Pathology, 199, 411-417 (2003)
Non-patent document No. 6: J. Biol. Chem., 278, 30142-30147 (2003)

### Disclosure of the Invention

### (Problems to be Solved by the Invention)

An object of the present invention is to provide a novel examination method for accurately evaluating the effectiveness of treatment with an anticancer drug targeting a tumor-associated factor receptor.

### (Means to Solve the Problems)

To solve the problems described above, the present inventor studied to find an examination method for accurately evaluating the effectiveness of trastuzumab (Herceptin^{™}) which is an anticancer drug targeting HER2/c-erbB-2 of tumor-associated factor receptor.

Currently, the effectiveness of trastuzumab (Herceptin^{™}) is evaluated by examining overexpression of HER2/c-erbB-2, for example, through the immunohistochemical analysis and the FISH method. In the above examination methods, proteins are determined to be positive only if stained in a cell membrane, but negative if stained only within the cytoplasm. Here, treatment with trastuzumab (Herceptin^{™}) to work effective needs that overexpressed HER2/c-erbB-2 is present functionally as a receptor on the cell membrane. Therefore, the inventor thought that, even if the examination methods as described above show the overexpression of c-erbB-2 gene and a large production of HER2/c-erbB-2, no HER2/c-erbB-2 anchored successfully on the cell membrane would suggest no expected effectiveness of treatment with trastuzumab (Herceptin™).

Further, a fairly diverse of cancers are recognized to overexpress HER2/c-erbB-2 at a high frequency. Currently, treatment with trastuzumab (Herceptin^{™}) has been put into practical use in breast cancer, but has given a too low proportion of effective cases for cancers in the other organs to be put into practical use. As the cause, the inventor estimated that overexpressed HER2/c-erbB-2 was unlikely to function on the cell membrane. Thus, the inventor has thought it to be quite useful for estimating therapeutic effects with trastuzumab (Herceptin^{™}) on an individual cancer that a substance, which is important for HER2/c-erbB-2 to function well on the cell membrane and interacts with the HER2/c-erbB-2 on the surface of and/or within the cell membrane, is examined beforehand to determine whether the substance is present or not in the cancer.

Thus, on cancer cases with overexpressed HER2/c-erbB-2 and treated with trastuzumab (Herceptin™), MUC4, that is, a substance which interacts with HER2/c-erbB-2 on the surface of and/or within cell membrane and makes the HER2/c-erbB-2 function effectively as a receptor on the cell membrane, was examined to clarify the relation between the expression state of the substance and the effectiveness of treatment with trastuzumab (Herceptin™).

As a result, it has found to be meaningful for an examination method for evaluating the usefulness of treatment with an anticancer drug targeting a tumor-associated factor receptor that the gene and/or the expressed product thereof of a substance which interacts with the receptor on the surface of and/or within a cell membrane is examined.The finding completes the present invention.

In summary, the present invention is composed of the followings:
1. An examination method conducted for the administration of an anticancer drug targeting a tumor-associated factor receptor, in order to evaluate usefulness of treatment with the anticancer drug, comprising, in addition to the examination of the gene and/or the expressed product thereof of the receptor, the examination of the gene and/or the expressed product thereof of a substance interacting with the receptor on the surface of and/or within the cell membrane.
2. The examination method according to the preceding clause 1, wherein the tumor-associated factor receptor is a cell growth factor receptor.
3. The examination method according to the preceding clause 2, wherein the cell growth factor receptor is an epidermal growth factor receptor or a receptor belonging to an epidermal growth factor receptor family.
4. The examination method according to the preceding clause 3, wherein the receptor belonging to the epidermal growth factor receptor family is HER2/c-erbB-2.
5. The examination method according to any one of the preceding clauses 1 to 4, wherein the substance interacting with the receptor on the surface of and/or within cell membrane is a glycoprotein.
6. The examination method according to the preceding clause 5, wherein the glycoprotein is a mucin.
7. The examination method according to the preceding clause 6, wherein the mucin is mucin 4 (MUC4).
8. The examination method according to any one of the preceding clauses 1 to 7, wherein the anticancer drug is an antibody to the receptor.
9. The examination method according to the preceding clause 8, wherein the antibody is a humanized monoclonal antibody.
10. The examination method according to the preceding clause 9, wherein the humanized monoclonal antibody is trastuzumab (Herceptin™).
11. A reagent for use in the examination method according to any one of the preceding clauses 1 to 10.
12. A reagent kit for use in the examination method according to any one of the preceding clauses 1 to 10.

### (Effects of the Invention)

The present invention can reliably predict the usefulness of treatment with an anticancer drug targeting a tumor-associated factor receptor. Further, that prediction allows planning of accurate therapeutic strategies and expectation of good effects on medical economy, such as avoidance of excessive dosing against cancers on which the treatment cannot be expected to effect.

### Description of the Preferred Embodiment

The present invention will be explained in detail below, and technical and scientific terms used herein have the same meanings as commonly understood by one of ordinary skill in the art to which this invention belongs unless defined otherwise.

The present invention is an examination method for evaluating usefulness of treatment with an anticancer drug conducted for administration of an anticancer drug targeting a tumor-associated factor receptor, comprising, in addition to the examination of the gene and/or the expressed product thereof of a receptor, the examination of the gene and/or the expressed product thereof of a substance interacting with the receptor on the surface of and/or within the cell membrane.

The tumor refers to a tissue whose cells break the harmony of an individual as a whole, though they are derived from the individual itself in vivo, to overgrow on their own accounts free from any other regulation. The tumor-associated factor refers to a factor involved in tumor formation. As the tumor-associated factor, a cytokine is preferred. The cytokine includes, but is not limited to, a growth factor, an interferon (IFN), a tumor necrosis factor (TNF), an interleukin (IL) and the like. Among the cytokines, the growth factor is more preferred. The growth factor includes, but is not limited to, an epidermal growth factor, a hepatocyte growth factors, a fibroblast growth factor, a platelet-derived growth factor, an insulin-like growth factor, a vascular endothelial growth factor and the like.

The receptor refers to a protein structure which allows the cell to recognize a particular chemical compound. Chemical mediators and various cell growth factors bind to their respective specific receptors present on the cell membrane, thereby allowing efficient intake into the cell and transmission of signals into the cell. The tumor-associated factor receptor is preferably a cell growth factor receptor, and more preferably an epidermal growth factor receptor and a receptor belonging to the same family. As the receptor belonging to the same family to the epidermal growth factor receptor, HER2/c-erbB-2 may be mentioned as a preferred example.

The substance interacting with a receptor on the surface of and/or within the cell membrane refers to a substance which interacts with a receptor on the surface of and/or within the cell membrane to play a role in expressing or enhancing the receptor's function. The substance which interacts with a receptor on the surface of and/or within the cell membrane is preferably a glycoprotein, and more preferably a mucin. Some of mucins are known as cancer antigens. Among the mucins, MUC4 is known to interact with HER2/c-erbB-2 protein within a cell, and thus it is mentioned as a preferred example.

The anticancer drug is a generic term of drugs for use against malignant tumors and inhibits expression of a cancer gene and/or function of the expressed product. The anticancer drug of the present invention targets a tumor-associated factor receptor. The definitions of these words have been explained above. An agent for molecular targeting therapy for cancers is illustrated as anticancer drugs. In particular, agents inhibiting signal transmission activities are representative, and so called tyrosine kinase inhibitors may be mentioned for example. Such agents include microorganism-derived Erbstatin, Lavendustin, Herbimycin A, Genistein; and chemically synthesized products such as benzylidenemalononitrile derivatives (patent publication No. JPH2-138238-A, Journal of Medical Chemistry, 32, 2344 (1989); Journal of Medical Chemistry 34, 1896 (1991)), α-cyanocinnamic acid amide derivatives (patent publication No. JPS63-222153-A), 3,5-diisopropyl-4-hydroxystyrene derivatives (patent publication No. JPS62-39522-A), 3,5-di-butyl-4-hydroxystyrene derivatives (patent publication No. JPS62-39523-A), and Erbstatin relative compounds (patent publication No. JPS62-277347-A).

The anticancer drug is preferably an antibody to a product involved in a signal transmission system, and more preferably a humanized monoclonal antibody. The product involved in a signal transmission system includes a receptor, thus an antibody to the receptor may be mentioned as an example. As the typical receptor, HER2/c-erbB-2 may be mentioned. Further, trastuzumab (Herceptin^{™}) which is a humanized monoclonal antibody to HER2/c-erbB-2 may preferably be mentioned. Besides them, as the agent for molecular targeting therapy, which is an anticancer drug of the present invention, ZD1839 (Iressa™), STI-571 and the like may be mentioned.ZD1839 (Iressa^{™}) is an inhibitor against the tyrosine kinase activity of EGFR, and STI-571 is an inhibitor against the tyrosine kinase activity of BCR-Ab1 and c-kit.

The cancer typically refers to a physiological condition of a mammal, characterized in that a cell grows out of regulation. Examples of the cancer include, but not limited to, carcinomas, lymphoma, blastoma, sarcoma, melanoma and leukemia. More particular examples of such cancer include squamous cell carcinoma, small cell lung cancer, non-small cell lung cancer, adenocarcinoma in lung, squamous carcinomas in lung, peritoneal cancer, hepatocellular carcinoma, gastrointestinal cancer, pancreas cancer, glioblastoma, endocervical carcinoma, non-small cell lung carcinoma, ovarian cancer, liver cancer, bladder cancer, hepatic cancer, breast cancer, colon cancer, colorectal cancer, endometrial carcinoma or uterine cancer, salivary gland cancer, renal cancer, prostate cancer, vulva cancer, thyroid cancer, and various type head and neck cancer.

As the gene encoding a product involved in signal transmission system, a gene directing a cell to get cancerous may be mentioned. Such gene includes, but not limited to, a gene having a growth factor function such as sis, int-2 and hst; a gene having a receptor-typed tyrosine kinase function such as erbB, erbB-2/neu, ros, fms, kit and ret; a gene having a nonreceptor-typed tyrosine kinase function such as src, yes, fgr, lck, fps/fes and ab1; a gene having a serine/threonine kinase function such as c-raf; the other gene like ras, bcl, int-1 and crk; and an intranuclear protein such as myc, fos, jun and erbA. Substances inhibiting the functions of some of these genes or functions of the expressed products thereof have been developed and used for pharmaceutical purposes. As the typical and preferred example, trastuzumab (Herceptin^{™}) used as an antibody agent against HER2/c-erbB-2 which is the gene product of erbB-2/neu may be mentioned.

As the analyte sample capable for use in the present invention, any tissue sample from a subject can be used. The tissue sample means an assembly of homologous cells obtained from the tissue of a subject or patient, and preferably contains a nucleated cell having a chromosomal substance. The tissue sample may contain components such as preservative, anticoagulant, buffer, fixative, nutrient and antibiotics, though they are not essentially found in a natural tissue. Examples of the tissue sample to be used include, but are not limited to, breast, prostate gland, ovary, colon, lung, endometrial, stomach, salivary gland, or spleen. The tissue sample can be obtained by various methods including, but are not limited to, surgical resection, aspiration, or biopsy. The tissue may be fresh or frozen.

The tissue sample can be fixed by a conventional method. A fixative can be decided to stain the tissue histologically or to analyze the tissue by other method. Further, a fixation period depends on the size of the tissue sample and a fixative to be used.
Usually, tissue samples are firstly fixed, and then dehydrated in an ascending alcohol series, and impregnated to embed in paraffin or the other sectioning medium for sectioning a tissue sample. Alternatively, a tissue is sectioned, and then the obtained sections are fixed. For example, tissue samples may be embedded in paraffin and processed by a conventional method. Once tissue samples are embedded, the samples may be sectioned with a microtome and the like. Once sectioned, the sections may be mounted onto a slide glass with several standard methods.

When paraffin is used as an embedding material, tissue sections are usually deparaffinized, and hydrated with water again. Tissue sections can be deparaffinized with some conventional standard methods. For example, xylene and a mild descending series of alcohol can be used.

As the examination method of genes and/or the expressed products thereof, a genetic method and an immunological method may be mentioned. Further, the present invention includes the combination of the genetic method and the immunological method, and further combinations with other examination methods.

The genetic method is a method to examine gene amplification, and conventional methods such as FISH, RT-PCR, and Southern blotting are used. Here, gene amplification means that there exist one or more additional genetic replicas of a gene encoding a tumor antigen of a complement for staining. Gene amplification may cause overexpression of protein. Probes for use in the genetic method may be oligonucleotides or polynucleotides of RNAs or DNAs.Probes may have a sufficient complementarity to the target nucleic acid sequence of a subject so as to produce a stable and specific binding with the target nucleic acid probe. The level of homology necessary for stable hybridization will be changed by stringencies of a hybridization medium and/or a wash medium. The selection of probe depends on the nature of a target gene.

The immunological method is a method for examining the expressed product of a gene with the antibody, and conventional methods such as immunohistochemical analysis and EIA are used. The antibody for use in the immunological method includes, but is not limited to, a monoclonal antibody, a polyclonal antibody and the fragments thereof.

The method described below is an example of the immunohistochemical staining using an antibody which specifically recognizes HER2/c-erbB-2.Tissue sections prepared by a common method and embedded in paraffin are deparaffinized with a xylene solution and a descending series of alcohol solution, and then washed with distilled water. Next, to remove endogenous peroxidase activity, the sections are reacted with a methanol containing 0.3% hydrogen peroxide at room temperature for 30 minutes, and then washed three times with 0.1M PBS for 5 minutes. Then, to inhibit nonspecific reactions, the sections are incubated with 0.1M PBS comprising 2 to 10% normal serum at room temperature for 30 to 60 minutes, followed by incubation with primary antibody recognizing HER2/c-erbB-2 at room temperature for an hour or at 4°C through day and night. Afterwards, the sections are washed three times with 0.1M PBS for 5 minutes, followed by incubation with secondary antibody labeled with peroxidase at room temperature for 30 minutes. Next, the sections are washed three times with 0.1M PBS for 5 minutes, then incubated with DAB reaction solution (10 mg of DAB tetrahydrochloride, 50 ml of PBS, 50µl of 1.5% H₂O₂) for 3 to 10 minutes to color, and then washed with flowing water for 10 minutes to terminate the color developing reaction.

In one embodiment of the present invention using the method for examining a gene of a tumor-associated factor receptor and/or the expressed product thereof, treatment with an anticancer drug targeting the receptor can be evaluated to be useful for a case wherein, in addition to the gene and/or the expressed product thereof of the receptor, the gene and/or the expressed product thereof of a substance which interacts with the receptor on the surface of and/or within the cell membrane is detected in an analyte sample.

As one example, HER2/c-erbB-2 is overexpressed not only in breast cancer but also in many cancers, but it is examined to make a practice of the treatment only for breast cancer. This is because the treatment has shown low effectiveness in high HER2/c-erbB-2 expressing cancers except breast cancer. For example, in colon cancer, HER2/c-erbB-2 is overexpressed at a high frequency, but MUC4 is found to be basically negative in colon epithelium including the tumor's, causing the treatment hardly to exhibit effectiveness. However, some cancers of MUC4 positive organs express HER2/c-erbB-2 at a high level. From cases of those cancers, a case which expresses both MUC 4 and HER2/c-erbB-2 is selected to treat with trastuzumab (Herceptin™).The case can be evaluated to be likely treated by high effectiveness. Therefore, the combination of the examinations for MUC4 and HER2/c-erbB-2 can broaden an applicable range for treatment with trastuzumab (Herceptin™).

Further, the present invention, in one embodiment, includes a reagent and a reagent kit capable for use in the examination method of the present invention. The reagent and the reagent kit may be provided as product forms, and the products comprise containers, optionally labels, and package inserts.

### (Example)

In the following, the present invention will be explained in more detail with reference to an example, but it is not intended to limit the technical scope of the present invention.

### EXAMPLE 1

With cases where HER2/c-erbB-2 were detected in tissues of breast cancer patients by immunohistochemical staining described above, the relation between therapeutic effects with trastuzumab (Herceptin^{™}) and stainability of MUC4 shown by immunohistochemical analysis was studied.
As a result, such results as shown in Table 1 were obtained. Among six cases which had an HER2/c-erbB-2 score of (++) or more, MUC4 positive were three wherein one was (+++) and two were (++).From these results, it was revealed that there were no mutual relations between HER2/c-erbB-2 and MUC4 expressions. Therefore, it was suggested to be meaningful to examine HER2/c-erbB-2 and MUC4 respectively.
Among three HER2/c-erbB-2 positive (+++) cases, one completely remitted case was MUC4 positive, one case showing no or poor effect was MUC4 negative, and one case rejected treatment with trastuzumab (Herceptin™).Both two HER2/c-erbB-2 positive (++) cases were MUC4 positive, one example of which was treated with trastuzumab (Herceptin^{™}) to show complete remission, although another was not treated with trastuzumab (Herceptin^{™}) because it was on an early stage.
From the facts above, it was revealed that a parallel relation did not always exist between MUC4 and HER2/c-erbB-2 expressions, and cases of MUC4 positive and HER2/c-erbB-2 positive (++) or more showed good responses to trastuzumab (Herceptin™), while cases of MUC4 negative showed poor effects of treatment with trastuzumab (Herceptin^{™}) though they were HER2/c-erbB-2 positive. Therefore, it was found that therapeutic effects with trastuzumab (Herceptin^{™}) can be predicted by the combination of the examinations for HER2/c-erbB-2 and MUC4.

**[Table 1]**

| Type of Breast Cancer | HER2/c-erbB-2 | MUC - 4 | Therapeutic Effects with Trastuzumab (Herceptin^{™}) |
|---|---|---|---|
| Papillotubular Carcinoma | ( - ) | ( - ) | Not Adapted |
| Papillotubular Carcinoma | ( - ) | ( + ) | Not Adapted |
| Papillotubular Carcinoma | ( - ) | ( - ) | Not Adapted |
| Scirrhous Carcinoma | ( - ) | ( - ) | Not Adapted |
| Scirrhous Carcinoma | (+++) | (-) | Poorly effective, thus the patient rejected the continuation of therapy and died. |
| Papillotubular Carcinoma | (+++) | ( - ) | Not effective. Note: after effects were shown by tamoxifen and Herceptin^{™}, tamoxifen was eliminated, causing the disappearance of effects. |
| Papillotubular Carcinoma | ( +++ ) | ( + ) | Completely Remitted |
| Papillotubular Carcinoma | ( +++ ) | ( - ) | Not Effective |
| Papillotubular Carcinoma | ( ++ ) | ( + ) | Completely Remitted |
| Papillotubular Carcinoma | ( ++ ) | ( + ) | Not Treated |
| Scirrhous Carcinoma | ( + ) | ( - ) | Not Treated |
| Papillotubular Carcinoma | ( + ) | ( - ) | Not Treated |
| Papillotubular Carcinoma | ( + ) | ( + ) | Not Treated |

### Industrial Applicability

The present invention can reliably predict the usefulness of treatment with an anticancer drug targeting a tumor-associated factor receptor, thereby to improve significantly the reliability for screening and selecting proper subjects to be treated with the anticancer drug. The prediction allows planning of accurate therapeutic strategies and broadening an applicable range for treatment with the anticancer drug. Further, the prediction allows expectation of good effects on medical economy, such as avoidance of excessive dosing against cancers on which the treatment cannot be expected to effect.

## Claims

1. An examination method conducted for the administration of an anticancer drug targeting a tumor-associated factor receptor, in order to evaluate usefulness of treatment with the anticancer drug, comprising, in addition to the examination of the gene and/or the expressed product thereof of the receptor, the examination of the gene and/or the expressed product thereof of a substance interacting with the receptor on the surface of and/or within the cell membrane.

2. The examination method according to Claim 1, wherein the tumor-associated factor receptor is a cell growth factor receptor.

3. The examination method according to the Claim 2, wherein the cell growth factor receptor is an epidermal growth factor receptor or a receptor belonging to an epidermal growth factor receptor family.

4. The examination method according to Claim 3, wherein the receptor belonging to the epidermal growth factor receptor family is HER2/c-erbB-2.

5. The examination method according to any one of Claims 1 to 4, wherein the substance interacting with the receptor on the surface of and/or within cell membrane is a glycoprotein.

6. The examination method according to Claim 5, wherein the glycoprotein is a mucin.

7. The examination method according to Claim 6, wherein the mucin is mucin 4 (MUC4).

8. The examination method according to any one of Claims 1 to 7, wherein the anticancer drug is an antibody to the receptor.

9. The examination method according to Claim 8, wherein the antibody is a humanized monoclonal antibody.

10. The examination method according to Claim 9, wherein the humanized monoclonal antibody is trastuzumab (Herceptin™).

11. A reagent for use in the examination method according to any one of Claims 1 to 10.

12. A reagent kit for use in the examination method according to any one of Claims 1 to 10.
